(19) Europäisches Patentamt
European Patent Office
Office européen des brevets

(11) Veröffentlichungsnummer: **0 078 922**
**B1**

(12) # EUROPÄISCHE PATENTSCHRIFT

(45) Veröffentlichungstag der Patentschrift: **23.01.85**

(51) Int. Cl.⁴: **C 07 C 39/14**, C 07 C 50/10, C 07 C 69/157, C 07 C 37/00, C 07 C 46/00, C 07 C 67/08, C 07 C 67/29

(21) Anmeldenummer: **82109284.8**

(22) Anmeldetag: **07.10.82**

(54) **Verfahren zur Herstellung der Vitamine K3 und K4 sowie von deren Derivaten.**

(30) Priorität: **19.10.81 DE 3141443**

(43) Veröffentlichungstag der Anmeldung: **18.05.83 Patentblatt 83/20**

(45) Bekanntmachung des Hinweises auf die Patenterteilung: **23.01.85 Patentblatt 85/4**

(84) Benannte Vertragsstaaten: **BE CH DE FR GB IT LI NL**

(56) Entgegenhaltungen:
**SU - A - 540 859**

**Chemische Berichte, Band 90, Nr. 9, 1957, VERLAG CHEMIE, Weinheim/Bergstr., F. WEYGAND et al. "Synthesen von alpha-Diacylbenzolen, Phthaliden und Naphthochinonen", Seiten 1879-1895**
**Chemisches Zentralblatt, Band 137,Nr. 21, 1966, Berlin, R.K. HILL et al. "Eine direkte Methode für den Aufbau von Benzolringen", Referat Nr. 0932**

(73) Patentinhaber: **BASF Aktiengesellschaft, Carl-Bosch-Strasse 38, D-6700 Ludwigshafen (DE)**

(72) Erfinder: **Kiefer, Hans, Dr., Im Sandgarten 5, D-6706 Wachenheim (DE)**

EP 0 078 922 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Beschreibung

Die vorliegende Erfindung betrifft ein neues Verfahren zur Herstellung der Vitamine $K_3$ und $K_4$ sowie von deren Derivaten. Diese Verbindungen haben die folgenden Formeln I bis IIIb:

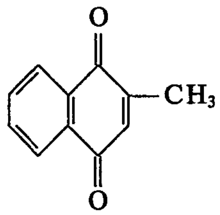

(I) Vitamin $K_3$; Menadion; 2-Methylnaphtho-1,4-chinon

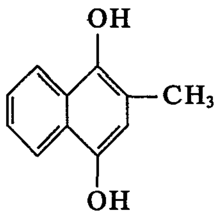

(IIa) Vitamin $K_4$; Menadiol; 2-Methyl-1,4-dihydroxynaphthalin

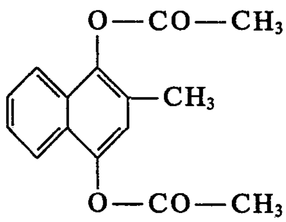

(IIb) Menadioldiacetat

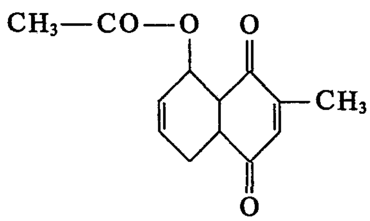

(IIIa) 2-Methyl-8-acetoxy-1,4-dioxo-1,4,4a,5,8,8a-hexahydronaphthalin

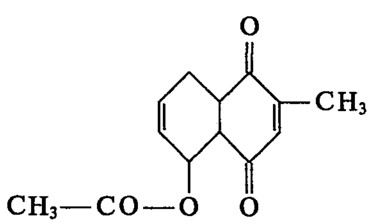

(IIIb) 2-Methyl-5-acetoxy-1,4-dioxo-1,4,4a,5,8,8a-hexahydronaphthalin

Außerdem betrifft die Erfindung die neuen Zwischenprodukte IIIa und IIIb.

Die antihämorrhagischen Vitamine der K-Gruppe (»Koagulationsvitamine«), darunter die Vitamine $K_3$ (I) und $K_4$ (IIa) sowie das Diacetat des Vitamins $K_4$ (IIb) dienen bekanntermaßen als blutungshemmende Arzneimittel und haben darüber hinaus große Bedeutung für die Tierernährung.

Unter den verschiedenen Methoden zur Herstellung von I (vgl. »Vitamine«, VEB Gustav Fischer Verlag, Jena, 1965, Seite 1050 f) hat sich bisher nur die Oxidation von 2-Methylnaphthalin mit Chromschwefelsäure durchgesetzt, obwohl die Ausbeute bei diesem Verfahren nur 20 bis 40% beträgt und obwohl das Arbeiten mit Chromschwefelsäure erhebliche verfahrenstechnische Schwierigkeiten bedingt. Da IIa bisher durch Reduktion von I hergestellt wird und IIb durch Acetylierung von IIa, waren IIa und IIb ebenfalls nur auf dem unbefriedigenden Weg über die Oxidation des 2-Methylnaphthalins zugänglich.

Aus Houben—Weyl, Band VII/3a, 1977, S. 80 f, sind verschiedene Naphthochinon-Synthesen durch Diels-Alder-Reaktion eines 1,4-Diens mit Benzochinon oder einem Benzochinonderivat, darunter dem Methylbenzochinon, bekannt. Im einzelnen handelt es sich dabei um folgende Reaktionen:

— Umsetzung von Butadien mit Benzochinon und anschließende Oxidation mit $CrO_3$; dieses Verfahren ist langwierig und umständlich und erfordert überdies die Verwendung des verfahrenstech-

nisch nachteiligen $CrO_3$;

— Umsetzung von 1,4-Diacetoxybutadien mit Benzochinon; diese Reaktion erfordert ein 4tägiges Erhitzen der Reaktionspartner und kommt deshalb für technische Zwecke ebenfalls nicht in Betracht;
— Umsetzung von Cyclohexa-1,3-dien und 2-Methylbenzochinon, Isomerisierung des Diels-Alder-Adduktes mit $HBr/H_2O$ zum entsprechenden Hydrochinonderivat, Oxidation der letztgenannten Verbindung mit $FeCl_3$ und thermische Eliminierung von Ethylen zu I; auch dieser Weg ist evident umständlich und daher für eine technische Synthese ungeeignet;
— Umsetzung von Crotonaldehyd mit Ethoxy-Mg-Br zum Butadien-1-oxy-Mg-Br, dessen Addition an Methylbenzochinon, Eliminierung von MgBrOH und Oxidation des Hydrochinons zu I; abgesehen davon, daß auch das Arbeiten mit magnesiumorganischen Verbindungen umständlich und unwirtschaftlich ist, sind die Ausbeuten bei dieser Reaktion unbefriedigend.

Der Erfindung lag daher die Aufgabe zugrunde, die wichtigen Verbindungen I, IIa und IIb auf einfachere und wirtschaftlichere Weise zugänglich zu machen als bisher.

Demgemäß wurde gefunden, daß man Vitamin $K_3$ (I), Vitamin $K_4$ (IIa) und das Diacetat des Vitamins $K_4$ (IIb)

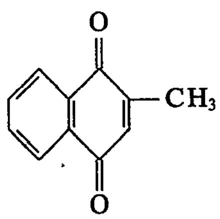

(I)

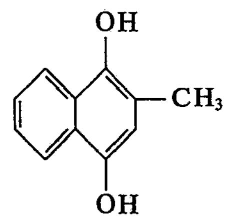

(IIa)

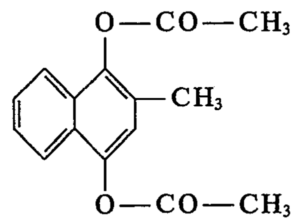

(IIb)

sowie die Vorstufen IIIa und IIIb dieser Verbindungen

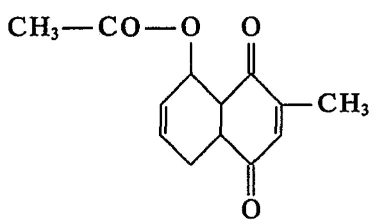

(IIIa)

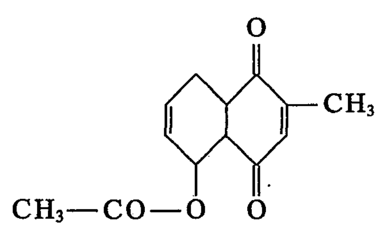

(IIIb)

in bemerkenswerten Reaktionsfolgen erhält, wenn man

a) 1-Acetoxybuta-1,3-dien (IV) mit 2-Methylbenzo-1,4-chinon (V) bei 20 bis 100°C zu einem Gemisch aus IIIa und IIIb umsetzt und diese Verbindungen wie üblich isoliert oder im Gemisch oder nach ihrer Isolierung
b) zur Herstellung von IIa auf bis zu 200°C erhitzt und/oder mit einer nicht-oxidierenden Säure behandelt,
c) zur Herstellung von IIb zusammen mit Acetanhydrid auf 50 bis 150°C erhitzt oder
d) zur Herstellung von I zusammen mit einem Oxidationsmittel auf 20 bis 100°C erhitzt.

Dieses Verfahren verläuft in allen Reaktionsschritten unerwartet glatt und führt zu Ausbeuten von über 80%, bezogen auf das eingesetzte Methylbenzochinon. Im Hinblick auf den geschilderten Stand der Technik ist die leichte Desacetylierung der Zwischenverbindungen IIIa und IIIb besonders überraschend.

Die Ausgangsverbindung IV, das 1-Acetoxy-buta-1,3-dien, ist bekannt und durch Umsetzung von Crotonaldehyd mit Acetanhydrid leicht und in guten Ausbeuten erhältlich (vgl. z. B. J. Org. Chem., Band 21 [1956], S. 330). Es hat sich erwiesen, daß die trans-Form von IV besonders glatt mit V zu IIIa und IIIb reagiert.

Die ebenfalls bekannte Ausgangsverbindung V, das Methylbenzo-1,4-chinon, kann vorteilhaft durch Oxidation von o-Toluidin mit Braunstein hergestellt werden.

Der Reaktionsschritt (a), eine Diels-Alder-Addition von IV an V, gelingt ohne Schwierigkeiten, und zwar vorzugsweise bei Temperaturen von 40 bis 80°C. Arbeitet man bei Temperaturen über 80°C, so findet bereits eine teilweise Desacetylierung zu IIa statt, was wirtschaftlich insofern nicht günstig ist,

als IIa unter Verbrauch von V zu I oxidiert wird, d. h. V fungiert hier als relativ teures Oxidationsmittel, wo ein billigeres Oxidationsmittel den gleichen Zweck erfüllt.

Zweckmäßigerweise nimmt man die Umsetzung (a) in Gegenwart eines Lösungsmittels vor. Geeignet sind alle diejenigen inerten organischen Flüssigkeiten, in denen sich sowohl IV als auch V lösen, also beispielsweise Dimethylformamid, Toluol, Xylol, N-Methylpyrrolidon, Acetonitril und Ethylmethylketon. Besonders empfehlen sich $C_5-C_8$-Alkane und Cycloalkane wie Cyclopentan und Cyclohexan, da die entstehenden Verbindungen IIIa und IIIb hierin unlöslich sind, sich als Öl abscheiden und somit unerwünschten Nebenreaktionen entzogen werden.

IV wird vorzugsweise in einem 10—20%igen molaren Überschuß über V eingesetzt, und die Menge des Lösungsmittels liegt in der Regel zwischen 2 und 10 kg pro kg des Einsatzstoffgemisches.

Da die Reaktion (a) bei Normaldruck abläuft, besteht in der Regel kein Grund, unter vermindertem oder erhöhtem Druck zu arbeiten. Erhöhter Druck kann jedoch zweckmäßig sein, wenn man ein tiefsiedendes Lösungsmittel wie Butan oder Methylenchlorid verwendet.

Man erhält normalerweise ein Gemisch aus etwa 30% IIIa und 70% IIIb, wobei beide Verbindungen nach den bisherigen Beobachtungen stereoeinheitlich sind und wobei IIIb die größere Kristallisationstendenz aufweist und in den Folgestufen etwas leichter reagiert.

In der Praxis ist es jedoch häufig am einfachsten, für die Folgereaktionen das aus IIIa und IIIb bestehende Reaktionsgemisch einzusetzen, wie es bei der Diels-Alder-Reaktion (a) anfällt.

Zur Herstellung von IIa gemäß Verfahrensschritt (b) genügt es, die Verbindungen IIIa, IIIb oder deren Gemisch 1 bis 5 Stunden lang auf 100 bis 200°C zu erhitzen. Diese Reaktion kann durch Mitverwendung einer Säure, z. B. Essigsäure, beschleunigt werden, wodurch sich entsprechend auch die Temperatur senken läßt. Verwendet man eine starke nicht-oxidierende Säure wie Salzsäure, wäßrige Schwefelsäure, Phosphorsäure oder einen sauren Ionenaustauscher, so kann sich das Erhitzen gänzlich erübrigen, d. h. man arbeitet in diesem Falle vorzugsweise bei Raumtemperatur oder leicht erhöhter Temperatur (etwa bis zu 60°C). Es hängt von den wirtschaftlich-technischen Gegebenheiten ab, welcher dieser Möglichkeiten — Erhitzen ohne Säure, mäßiges Erhitzen mit Säure oder Säurebehandlung ohne Erhitzen — man den Vorzug gibt. Die Menge der Säuren kann in einem weiten Bereich, etwa von 0,01—10 mol pro Mol IIIa bzw. IIIb, variieren.

Hinsichtlich des Drucks gelten die gleichen Überlegungen wie für den Reaktionsschritt (a), d. h. in der Regel arbeitet man bei Normaldruck.

Möchte man gemäß Verfahrensschritt (c) die Verbindungen IIb herstellen, so gelten die gleichen Bedingungen wie für den Verfahrensschritt (b) mit dem Unterschied, daß man die Reaktion in Gegenwart von 2 bis 6 mol Acetanhydrid pro Mol der Ausgangsverbindung IIIa und/oder IIIb vornimmt. Diese Reaktion kann durch katalytische Mengen Acetylchlorid beschleunigt werden. Um eine Ausbeuteverminderung durch unerwünschte Oxidationen zum Chinon zu vermeiden, kann es sich empfehlen, die Reaktion in Gegenwart von etwas Zinkstaub vorzunehmen.

Ähnlich verhält es sich beim Verfahrensschritt (d), der Herstellung von I aus IIIa und/oder IIIb. In diesem Falle verwendet man zweckmäßigerweise 2 bis 8 mol eines Oxidationsmittels pro Mol IIIa bzw. IIIb mit. Geeignete Oxidationsmittel sind beispielsweise Wasserstoffperoxid sowie vor allem Natriumnitrit und Salpetersäure.

Die Mitverwendung von Lösungsmitteln in den Verfahrensschritten (b) bis (d) ist nicht erforderlich, kann aber im Hinblick auf eine gleichmäßigere Reaktionsführung sowie im Hinblick auf die Aufarbeitung der Reaktionsgemische zweckmäßig sein. Als Lösungsmittel kommen z. B. Benzol, Toluol, Xylol und Essigsäure in Betracht.

Die Aufarbeitung der Reaktionsgemische auf die Verfahrensprodukte bietet keine grundsätzlichen Probleme und kann daher wie üblich vorgenommen werden. Das gleiche gilt für die Reindarstellung dieser Verbindungen.

## Beispiel 1

### Herstellung der Verbindungen IIIa und IIIb

Eine Mischung aus 122 g (1 mol) Methyl-p-benzochinon und 400 ml n-Heptan wurde im Laufe einer Stunde mit 135 g (1,2 mol) 1-Acetoxybuta-1,3-dien versetzt, wonach das Reaktionsgemisch noch 4 Stunden bei 60°C gehalten wurde. Beim Abkühlen auf Raumtemperatur schied sich eine ölige Phase ab, die abgetrennt und zweimal mit je 100 ml n-Heptan gewaschen wurde. Anschließend wurden das restliche Lösungsmittel sowie das überschüssige 1-Acetoxybuta-1,3-dien unter vermindertem Druck bei 60°C entfernt. Das zurückbleibende Öl, welches die beiden Isomeren IIIa und IIIb nach NMR-Analyse im Verhältnis 30 : 70 enthielt, erstarrte nach einiger Zeit zu farblosen Kristallen vom Smp. 73—90°C; Ausbeute 96%, bezogen auf eingesetztes Methylbenzochinon.

Durch Umkristallisation des Gemisches aus 800 ml Methanol wurde das bevorzugt kristallisierende Isomere IIIb in reiner Form gewonnen; Smp. 99°C, Ausbeute rund 60%, bezogen auf eingesetztes Methylbenzochinon.

Beispiel 2

Herstellung der Verbindung IIa

122 g (1 mol) Methylbenzochinon und 135 g (1,2 mol) 1-Acetoxybuta-1,3-dien wurden zunächst analog Beispiel 1 zu dem Isomerengemisch aus IIIa und IIIb umgesetzt, wobei statt des Heptans 300 ml o-Xylol als Lösungsmittel verwendet wurden.

Die so erhaltene xylolische Lösung wurde sodann durch Einleiten von Stickstoff vom Luftsauerstoff befreit, worauf sie 4 Stunden lang unter Rückflußkühlung (144° C) erhitzt wurde.

Danach wurde das Reaktionsgemisch abgekühlt, worauf IIa als farbloser Niederschlag ausfiel; Smp. 177—178° C; Ausbeute 81%, bezogen auf eingesetztes Methylbenzochinon.

Beispiel 3

Herstellung der Verbindung IIa

23,4 g (0,1 mol) IIIb wurden unter Rühren in 100 ml 20gew.-%ige Salzsäure (rund 0,6 mol HCl) eingetragen. Das Gemisch wurde auf 30—40° C erwärmt, worauf IIIb in Lösung ging. Unmittelbar anschließend schieden sich farblose Kristalle von IIa ab.

Die Abscheidung des IIa wurde durch Zugabe von 100 ml Wasser vervollständigt. Die Ausbeute an IIa, bezogen auf eingesetzten IIIb, betrug 95%.

Mit gleichem Erfolg wurden 100 ml 50gew.-%ige Phosphorsäure anstelle der Salzsäure verwendet.

Beispiel 4

Herstellung von I

250 ml 30gew.-%ige wäßrige Salpetersäure (rund 0,7 mol $HNO_3$) wurden unter starkem Rühren bei 50—60° C im Laufe von 20 min mit 23,4 g (0,1 mol) des nach Beispiel 1 hergestellten Isomerengemisches aus IIIa und IIIb versetzt. Das Gemisch wurde noch eine Stunde bei 50—60° C nachgerührt und danach abgekühlt, wobei I in Form gelber Kristalle anfiel. Das Produkt wurde abfiltriert, mit Wasser gewaschen und getrocknet. GC-analytisch waren keine Verunreinigungen mehr nachweisbar; Smp. 105° C; Ausbeute 86%.

Beispiel 5

Herstellung von IIb

Analog Beispiel 1 wurde zunächst aus 122 g (1 mol) Methylbenzochinon und 123 g (1,1 mol) 1-Acetoxybuta-1,3-dien ein Isomerengemisch aus IIIa und IIIb hergestellt, wobei 400 ml reine Essigsäure als Lösungsmittel dienten.

Das so erhaltene Gemisch wurde anschließend unter Stickstoff mit 408 g (4 mol) Acetanhydrid und 5 g Acetylchlorid 4 Stunden lang unter Rückflußkühlung erhitzt. Danach wurde das Gemisch noch 30 min zusammen mit 2 g Zinkpulver erhitzt, filtriert und destillativ aufgearbeitet. Als Hauptfraktion fiel hierbei (0,01 mbar, 160° C) ein farbloses Öl an, welches kristallin erstarrte (Smp. 109° C).

Die Umkristallisation dieser Masse lieferte reines IIb in einer Ausbeute von 85%; Smp. 115° C.

Beispiel 6

Herstellung von I

Eine nach dem ersten Absatz von Beispiel 5 hergestellte essigsaure Lösung von IIIa und IIIb wurde 5 h zum Sieden erhitzt, bei 50—60° C mit einer Lösung aus 600 ml Wasser und 376 g (rund 5,5 mol) Natriumnitrit versetzt und eine Stunde bei dieser Temperatur gehalten, wobei I in Form eines gelben kristallinen Niederschlages ausfiel. Die übliche Aufarbeitung lieferte das reine I in 87%iger Ausbeute, bezogen auf eingesetztes Methylbenzochinon.

**Patentansprüche**

1. Verfahren zur Herstellung von Vitamin $K_3$ (I), Vitamin $K_4$ (IIa) und Vitamin $K_4$-Diacetat (IIb)

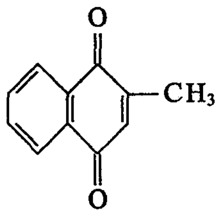

(I)

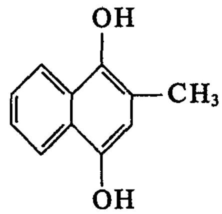

(IIa)

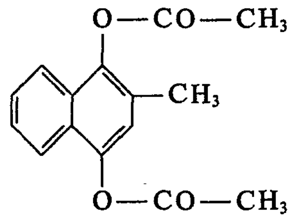

(IIb)

sowie den Vorstufen IIIa und IIIb dieser Verbindungen

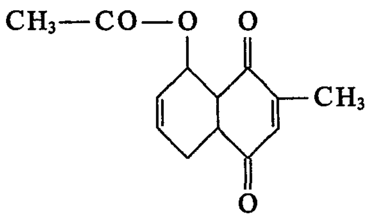

(IIIa)

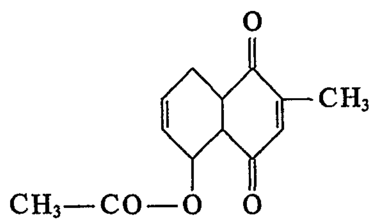

(IIIb)

dadurch gekennzeichnet, daß man

a) 1-Acetoxybuta-1,3-dien (IV) mit 2-Methylbenzo-1,4-chinon (V) bei 20 bis 100°C zu einem Gemisch aus IIIa und IIIb umsetzt und diese Verbindungen wie üblich isoliert oder im Gemisch oder nach ihrer Isolierung
b) zur Herstellung von IIa auf bis zu 200°C erhitzt und/oder mit einer nicht-oxidierenden Säure behandelt,
c) zur Herstellung von IIb zusammen mit Acetanhydrid auf 50 bis 150°C erhitzt oder
d) zur Herstellung von I zusammen mit einem Oxidationsmittel auf 20 bis 100°C erhitzt.

2. 2-Methyl-8-acetoxy-1,4-dioxo-1,4,4a,5,8,8a-hexahydronaphthalin (IIIa).

3. 2-Methyl-5-acetoxy-1,4-dioxo-1,4,4a,5,8,8a-hexahydronaphthalin (IIIb).

**Claims**

1. A process for the preparation of vitamin $K_3$ (I), vitamin $K_4$ (IIa) and vitamin $K_4$ diacetate (IIb)

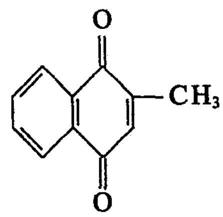

(I)

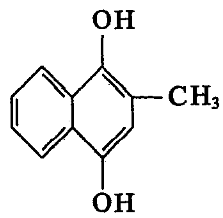

(IIa)

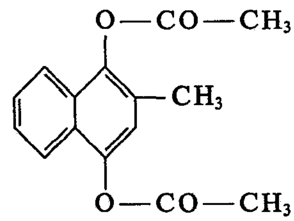

(IIb)

and of the intermediates IIIa and IIIb of these compounds

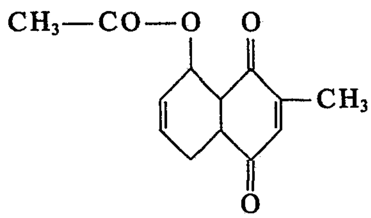

(IIIa)

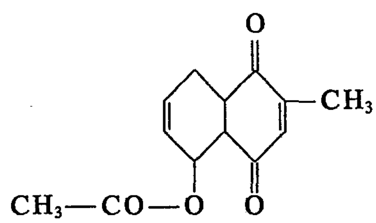

(IIIb)

wherein

a) 1-acetoxybuta-1,3-diene (IV) is reacted with 2-methylbenzo-1,4-quinone (V) at from 20 to 100° C to give a mixture of IIIa and IIIb and these compounds are isolated in a conventional manner or, as a mixture or after being isolated,
b) for the preparation of IIa, are heated to not more than 200° C and/or treated with a non-oxidizing acid,
c) for the preparation of IIb, are heated, together with acetic anhydride, to from 50 to 150° C, or
d) for the preparation of I, are heated, together with an oxidizing agent, to from 20 to 100° C.

2. 2-Methyl-8-acetoxy-1,4-dioxo-1,4,4a,5,8,8a-hexahydronaphthalene (IIIa).

3. 2-Methyl-5-acetoxy-1,4-dioxo-1,4,4a,5,8,8a-hexahydronaphthalene (IIIb).

**Revendications**

1. Procédé de préparation de la vitamine $K_3$ (I), de la vitamine $K_4$ (IIa) et du diacétate de vitamine $K_4$ (IIb)

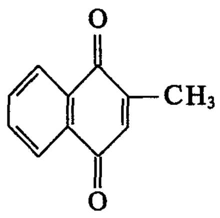 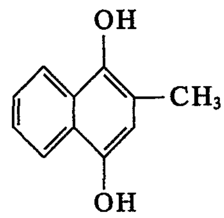 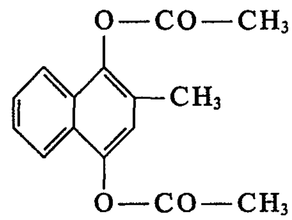

(I) (IIa) (IIb)

ainsi que des précurseurs IIIa et IIIb de ces composés

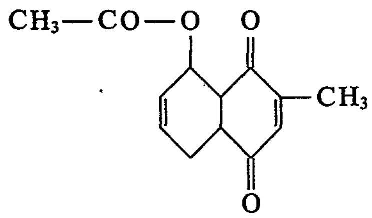 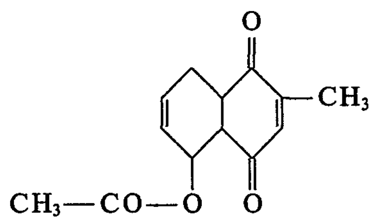

(IIIa) (IIIb)

caractérisé en ce que:

a) on fait réagir le 1-acétoxy-buta-1,3-diène (IV) et la 2-méthyl-benzo-1,4-quinone (V) entre 20 et 100°C pour obtenir un mélange de IIIa et de IIIb, ces composés étant isolés de manière usuelle ou utilisés en mélange ou après isolement
b) pour l'obtention de IIa par un chauffage jusqu'à 200°C et (ou) un traitement par un acide non oxydant
c) pour l'obtention de IIb par un chauffage entre 50 et 150°C en présence d'anhydride acétique
d) pour l'obtention de I par un chauffage entre 20 et 100°C en présence d'un agent d'oxydation.

2. Le 2-méthyl-8-acétoxy-1,4-dioxo-1,4,4a,5,8,8a-hexahydro-naphtalène (IIIa).

3. Le 2-méthyl-5-acétoxy-1,4-dioxo-1,4,4a,5,8,8a-hexahydro-naphtalène (IIIb).